# EUROPÄISCHE PATENTSCHRIFT

(11) **EP 4 675 112 B1**
(45) Veröffentlichungstag und Bekanntmachung des Hinweises auf die Patenterteilung: **20.05.2026**
(21) Anmeldenummer: 25186657.0
(22) Anmeldetag: 01.07.2025
(51) Int. Cl.: F16B 5/02, A61M 1/16

(54) **BAUTEILANORDNUNG FÜR EIN MEDIZINISCHES GERÄT**
COMPONENT ARRANGEMENT FOR A MEDICAL DEVICE
ENSEMBLE DE COMPOSANTS POUR UN APPAREIL MÉDICAL

(30) Priorität: 02.07.2024 DE 102024118737
(43) Veröffentlichungstag der Anmeldung: 07.01.2026
(73) Patentinhaber: B. Braun Avitum AG, 34212 Melsungen (DE)
(72) Erfinder: Knierim, Michael, 37242 Bad Sooden-Allendorf (DE)
(74) Vertreter: Patentanwälte Ruff, Wilhelm, Beier, Dauster & Partner mbB

(56) Entgegenhaltungen:
- DE-A1- 102007 048 298
- DE-A1- 102018 105 964
- DE-A1- 102021 207 384
- DE-B4- 102017 104 384

## Beschreibung

Die Erfindung betrifft eine Bauteilanordnung für ein medizinisches Gerät, insbesondere ein Dialysegerät.

Medizinische Geräte, wie beispielsweise Dialysegeräte, weisen in der Regel ein aus mehreren Bauteilen zusammengefügtes Gehäuse auf, wobei als Fügeverbindungen zwischen den Bauteilen oftmals Schraubverbindungen vorgesehen sind. Bei aus Kunststoff gefertigten Bauteilen finden üblicherweise sogenannte Kunststoff-Direktverschraubungen Anwendung. Solche Schraubverbindungen umfassen einen Schraubdom, in welchen eine Schraube mit selbstfurchendem Gewinde eingedreht ist.

Bei herkömmlichen medizinischen Geräten wird zur Befestigung eines Kunststoffbauteils an einer Blechschnittstelle der Schraubdom einer Direktverschraubung innerhalb des Kunststoffbauteils unter einem ebenen Blech realisiert. Dabei wird eine komplette Länge des Schraubdoms für die Direktverschraubung in einem im Sichtbereich für den Anwender optisch nicht ansprechenden, stark auftragenden Aufbau eingebracht.

Aus der DE 10 2017 104 384 B4 ist ein Bauteil mit einem Schraubdom zur Aufnahme einer Schraube bekannt, wobei der Schraubdom aus dem Bauteil herausragt und einstückig mit dem Bauteil verbunden ist. Der Schraubdom weist einen von einer Wandung umgebenen Hohlraum auf, der zur Einführseite der Schraube geöffnet ist. Der Schraubdom weist Versteifungen auf, zwischen denen eine partielle Schwächung der Wandung vorliegt.

Aus der DE 10 2007 048 298 A1 ist eine Befestigungsvorrichtung bekannt, aufweisend mindestens einen an einem Kunststoffgehäuse ausgebildeten Dom über welchen ein einstückig mit demselben ausgebildeter Stift übersteht, wobei das Ende des Stifts der Fixierung eines an der Befestigungsvorrichtung anzubringenden Elements dient, und der Stift einen Absatz aufweist, welcher als Anlagefläche für das an der Befestigungsvorrichtung anzubringende Element dient, wobei zumindest im zentralen Bereich des Stifts eine Aussparung oder Ausnehmung vorgesehen ist, welche sich bis unterhalb der durch den Absatz gebildeten Anlagefläche erstreckt.

Aufgabe der Erfindung ist es, eine Bauteilanordnung für ein medizinisches Gerät bereitzustellen, die Vorteile gegenüber dem Stand der Technik bietet. Insbesondere soll die Bauteilanordnung einen flachen Aufbau im Sichtbereich des Anwenders ermöglichen.

Diese Aufgabe wird durch das Bereitstellen einer Bauteilanordnung mit den Merkmalen des Anspruchs 1 gelöst. Vorteilhafte Weiterbildungen sind in den Unteransprüchen angegeben. Der Wortlaut der Ansprüche wird durch Bezugnahme zum Gegenstand der Beschreibung gemacht.

Die erfindungsgemäße Bauteilanordnung weist ein erstes Bauteil, ein zweites Bauteil und mindestens eine Schraube auf. Das erste Bauteil weist eine Innenseite, eine Außenseite und eine Aussparung auf. Die Aussparung ist zwischen der Innenseite und der Außenseite erstreckt. Das erste Bauteil weist weiter mindestens eine Schraubenaufnahme auf, die in der Aussparung angeordnet ist und in Richtung der Innenseite um einen axialen Abstand von der Außenseite zurückversetzt ist. Das zweite Bauteil weist einen Schraubdom mit einem längserstreckten Domschaft und einer Bohrung auf, wobei das zweite Bauteil wenigstens abschnittsweise an der Außenseite des ersten Bauteils angeordnet ist und der Domschaft axial in die Aussparung hineinragt. Die Schraube weist einen Schraubenschaft mit einem Gewinde und einen Schraubenkopf auf, wobei der Schraubenschaft in die Bohrung eingeschraubt ist und der Schraubenkopf axial auf der Schraubenaufnahme abgestützt ist, wodurch das erste Bauteil mit dem zweiten Bauteil verschraubt ist. Bei einer Ausgestaltung ragt der Domschaft axial durch die Aussparung hindurch über die Innenseite des ersten Bauteils hinaus. Bei einer weiteren Ausgestaltung ist der Schraubenschaft durch die Schraubenaufnahme in die Bohrung eingeschraubt. Bei einer weiteren Ausgestaltung handelt es sich bei dem ersten Bauteil und bei dem zweiten Bauteil jeweils um ein Gehäusebauteil des medizinischen Geräts. Das zweite Bauteil kann beispielsweise eine Frontplatte, eine Abdeckung oder eine Blende sein.

Die erfindungsgemäße Bauteilanordnung bietet unterschiedliche Vorteile gegenüber dem Stand der Technik. Insbesondere kann durch die geometrische Gestaltung des ersten Bauteils und des zweiten Bauteils eine im Vergleich zu etablierten Gestaltungsregeln schlankere oder auch flachere Ausführung des zweiten Bauteils erreicht werden.

In Ausgestaltung der Erfindung entspricht der axiale Abstand wenigstens im Wesentlichen einer axialen Länge des Domschafts. Hierdurch kann ein besonders flacher Aufbau erreicht werden. Dabei ist vorzugsweise ein Stirnende des Schraubdoms axial auf der Schraubenaufnahme abgestützt bzw. liegt an dieser an, sodass sich weder zwischen dem Stirnende des Schraubdoms und der Schraubenaufnahme noch zwischen der Außenseite des ersten Bauteils und einer Innenseite des zweiten Bauteils im Bereich außerhalb der Aussparung ein Axialspalt bildet. Bei einer Ausgestaltung ist der axiale Abstand größer als die Länge des Domschafts, wodurch sich zwischen dem Stirnende des Schraubdoms und der Schraubenaufnahme ein Axialspalt bildet. Der Axialspalt gewährleistet einen vorteilhaften Kraftfluss, indem der Schraubenschaft und der Schraubdom axial auf Zug beansprucht werden und der Schraubenkopf axial auf die Schraubenaufnahme drückt. Hierdurch wird das zweite Bauteil axial gegen die Außenseite des ersten Bauteils gezogen/gepresst.

In weiterer Ausgestaltung der Erfindung ist das erste Bauteil ein Blechbauteil und die mindestens eine Schraubenaufnahme ist an einer Lasche angeordnet. Dabei ist die Lasche durch einen abgekanteten Abschnitt des Blechbauteils ausgebildet. Hierdurch wird eine vereinfachte Fertigung ermöglicht. Bei einer Ausgestaltung kann die Lasche an einer Nibbelmaschine durch ein Laschenwerkzeug umgeformt sein.

In Ausgestaltung der Erfindung ist die Schraubenaufnahme mit mindestens einer Ausnehmung zur Aufnahme des Schraubenschafts versehen. Bei einer Ausgestaltung der Schraubenaufnahme ist die Ausnehmung radial geschlossen. Bei einer weiteren Ausgestaltung der Schraubenaufnahme ist die Ausnehmung radial offen. Die Ausnehmung kann eine Zylinderbohrung, ein Langloch oder dergleichen sein. Die Ausnehmung kann durch Bohren, Fräsen, Stanzen, Laserschneiden oder dergleichen in das erste Bauteil eingebracht sein.

In weiterer Ausgestaltung der Erfindung ist das erste Bauteil mit mindestens zwei Schraubenaufnahmen versehen, wobei die Schraubenaufnahmen an mindestens zwei Umfangsseiten der Aussparung ausgebildet sind. Bei einer Ausgestaltung sind die Schraubenaufnahmen an zwei gegenüberliegenden Umfangsseiten der Aussparung ausgebildet.

In weiterer Ausgestaltung der Erfindung weist das erste Bauteil mehrere Positionierelemente und das zweite Bauteil mehrere komplementäre Positionieraufnahmen auf. Dabei greifen die Positionierelemente jeweils in eine der Positionieraufnahmen formschlüssig ein, wodurch die beiden Bauteile in vorbestimmter Lage aneinander ausgerichtet sind. Hierdurch werden Montagefehler und Folgeschäden vermieden. Bei einer Ausgestaltung haben die Positionierelemente auch eine Haltefunktion. Hierdurch ergibt sich eine weiter vereinfachte Montierbarkeit. Bei einer Ausgestaltung weist das zweite Bauteil mehrere Positionierelemente und das erste Bauteil mehrere komplementäre Positionieraufnahmen auf.

In weiterer Ausgestaltung der Erfindung ist das zweite Bauteil aus einem Kunststoffmaterial gefertigt. Bei einer Ausgestaltung ist das zweite Bauteil spritzgegossen. Bei einer weiteren Ausgestaltung ist auch das erste Bauteil aus einem Kunststoffmaterial gefertigt, im Speziellen spritzgegossen.

In weiterer Ausgestaltung ist das Gewinde der Schraube selbstfurchend. Selbstfurchend meint, dass das Gewinde der Schraube beim Einschrauben in den Domschaft sein Gegengewinde selbst ausbildet, im Speziellen furcht und/oder schneidet. Das Gegenwinde muss daher nicht in einem gesonderten Fertigungsschritt in den Schraubendom eingebracht werden. Diese Ausgestaltung ist besonders dann vorteilhaft, wenn das zweite Bauteil aus einem/dem Kunststoffmaterial gefertigt ist.

In weiterer Ausgestaltung ist die Schraube mit einer Unterlegscheibe versehen, die form- und/oder kraftschlüssig mit einer Unterseite des Schraubenkopfs verbunden ist. Die Unterlegscheibe ist folglich axial zwischen dem Schraubenkopf und der Schraubenaufnahme angeordnet. Durch die Abmessungen und/oder Materialeigenschaften der Unterlegescheibe können die Eigenschaften der Schraubverbindung positiv beeinflusst werden.

Die Erfindung betrifft zudem ein medizinisches Gerät mit einer Bauteilanordnung gemäß der vorhergehenden Beschreibung. Im Speziellen handelt es sich bei dem medizinischen Gerät um ein Dialysegerät zur extrakorporalen Blutbehandlung.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Perspektivansicht eine Ausführungsform eines erfindungsgemäßen medizinischen Geräts in Form eines Dialysegeräts mit einem Gehäuse, das eine Ausführungsform einer erfindungsgemäßen Bauteilanordnung aufweist,
- Fig. 2: in teilweise abgeschnittener, schematischer Seitenansicht die erwähnte Bauteilanordnung des medizinischen Geräts nach Fig. 1,
- Fig. 3: eine längsgeschnittene Darstellung der Bauteilanordnung nach Fig. 2 im Bereich eines Schraubdoms,
- Fig. 4: in schematischer Perspektivansicht ein erstes Bauteil mit einer Ausführungsform einer Schraubenaufnahme und
- Fig. 5: in einer der Fig. 4 entsprechenden Darstellungsweise das erste Bauteil mit einer weiteren Ausführungsform einer Schraubenaufnahme.

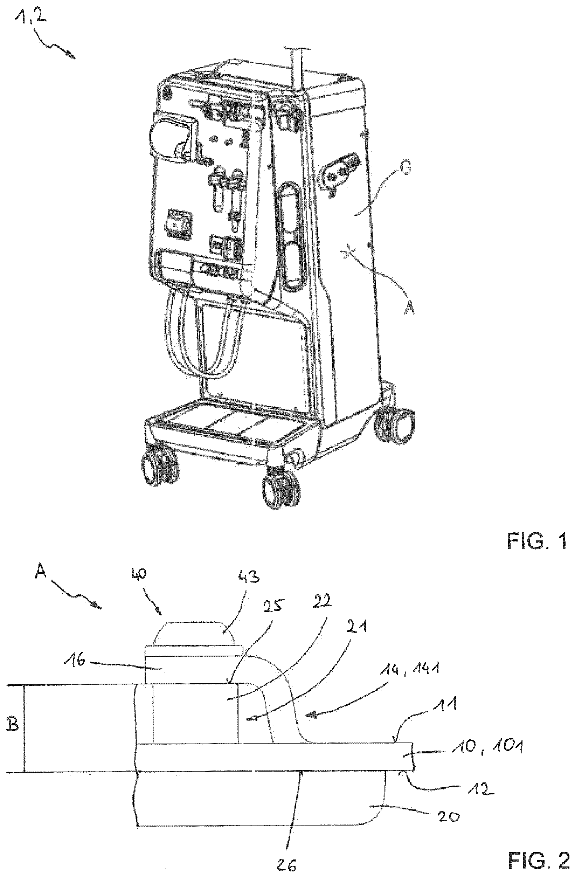

Gemäß Fig. 1 ist ein medizinisches Gerät 1 in Form eines Dialysegeräts 2 zur Verwendung bei einer extrakorporalen Blutbehandlung vorgesehen. Das Dialysegerät 2 weist eine der Fachperson grundsätzlich bekannte Funktion auf, so dass auf diesbezügliche Merkmale des Dialysegeräts 2 nicht weiter eingegangen zu werden braucht.

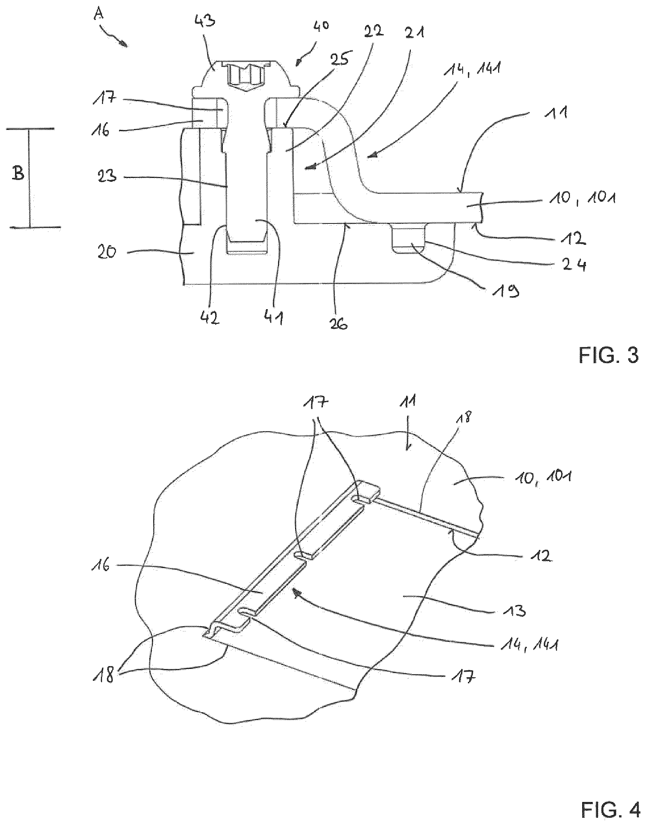

Das Dialysegerät 2 weist ein Gehäuse G auf. Das Gehäuse G umfasst eine Bauteilanordnung A (in Fig. 1 nicht ersichtlich), wie sie im Detail in den Fig. 2 und 3 gezeigt ist.

Die Bauteilanordnung A weist ein erstes Bauteil 10, ein zweites Bauteil 20 und eine Schraube 40 auf.

Das erste Bauteil 10 und das zweite Bauteil 20 sind auf noch näher beschriebene Weise miteinander verschraubt. Bei der Darstellung der Ausführungsform gemäß Fig. 2 ist nur eine Schraube gezeigt. Die Anzahl ist als rein exemplarisch zu verstehen und kann eine beliebige Zahl sein, beispielsweise zwei, vier oder sechs. Nachfolgend werden die weiteren Merkmale der Bauteilanordnung A unter Bezugnahme auf eine/die Schraube 40 erläutert. Für die weiteren, nicht gezeigten Schrauben gilt grundsätzlich das im Hinblick auf die Schraube 40 Gesagte sinngemäß.

Das erste Bauteil 10 weist eine Innenseite 11, eine Außenseite 12, eine Aussparung 13 und mindestens eine Schraubenaufnahme 16 auf. Die Außenseite 12 ist einer Umgebung zugewandt. Die Innenseite 11 ist einem Inneren des Gehäuses G zugewandt und/oder der Umgebung abgewandt. Die Aussparung 13 ist zwischen der Innenseite 11 und der Außenseite 12 erstreckt. Die Schraubenaufnahme 16 ist in der Aussparung 13 angeordnet und ist in Richtung der Innenseite 12 um einen axialen Abstand B von der Außenseite 12 zurückversetzt (siehe insbesondere Fig. 2).

Die in Fig. 4 gezeigte Schraubenaufnahme 16 erstreckt sich über eine gesamte Länge einer Umfangsseite 18 der Aussparung 13.

Bei nicht gezeigten Ausführungsformen erstreckt sich die eine Schraubenaufnahme 16 über eine Teillänge der Umfangsseite 18 der Aussparung 13.

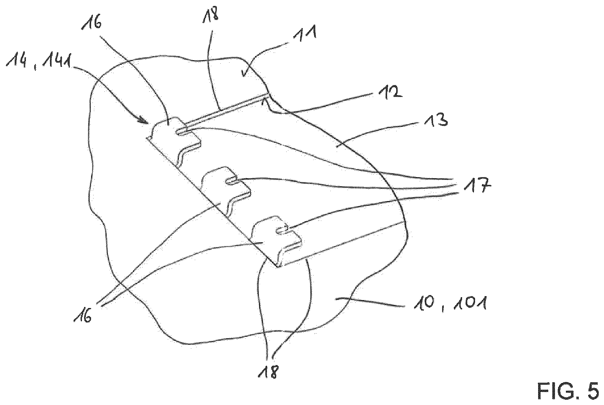

Die in Fig. 5 gezeigten Schraubenaufnahmen 16 erstrecken sich jeweils über eine Teillänge der Umfangsseite 18 der Aussparung 13 und sind gleichmäßig zueinander beabstandet.

Bei nicht gezeigten Ausführungsformen sind die Schraubenaufnahmen 16 ungleichmäßig zueinander beabstandet.

Dabei sind am ersten Bauteil 10 bei der in Fig. 4 gezeigten Ausführungsform insgesamt zwei, auf gegenüberliegenden Umfangsseiten 18 der Aussparung 13 angeordnete, Schraubenaufnahmen 16 und bei der in Fig. 5 gezeigten Ausführungsform insgesamt sechs, auf gegenüberliegenden Umfangsseiten 18 der Aussparung 13 angeordnete, Schraubenaufnahmen 16 vorhanden, wobei in den Figuren jeweils nur ein Teil der Schraubenaufnahmen 16 gezeigt ist. Die gezeigte Anzahl ist als rein exemplarisch zu verstehen.

Bei nicht gezeigten Ausführungsformen sind die Schraubenaufnahmen 16 an mindestens zwei, beispielsweise über Eck, aneinandergrenzenden Umfangsseiten 18 der Aussparung 13 ausgebildet.

Bei nicht gezeigten Ausführungsformen sind nur auf einer der Umfangsseiten 18 der Aussparung 13 eine oder mehrere Schraubenaufnahmen 16 angeordnet. Auf der der mindestens einen Schraubenaufnahme 16 gegenüberliegenden Umfangsseite 18 kann eine andere Befestigungsart vorgesehen sein. Dabei kann es sich zum Beispiel um eine andere Art der Verschraubung, um eine Nut-Feder-Verbindung oder dergleichen handeln.

Das zweite Bauteil 20 ist bei der gezeigten Ausführungsform aus einem Kunststoffmaterial K gefertigt. Die Fertigung erfolgt vorliegend per Spritzguss. Es sind auch andere Fertigungsweisen denkbar und möglich, beispielsweise eine additive Fertigung, im Speziellen per 3D-Druck, oder eine spanende Fertigung, zum Beispiel Fräsen.

Das zweite Bauteil 20 weist einen Schraubdom 21 mit einem längserstreckten Domschaft 22 und einer Bohrung 23 auf. Das zweite Bauteil 20 ist an der Außenseite 12 des ersten Bauteils 10 angeordnet. Dabei ragt der Domschaft 22 axial in die Aussparung 13 hinein und über die Innenseite 11 des ersten Bauteils 10 hinaus.

Die Schraube 40 weist einen Schraubenschaft 41 mit einem Gewinde 42 und einen Schraubenkopf 43 auf. Dabei ist der Schraubenschaft 41 durch die Schraubenaufnahme 16 hindurch in die Bohrung 23 eingeschraubt. Beim Einschrauben der Schraube 40 schneidet das vorliegend selbstfurchend gestaltete Gewinde 42 gleichsam ihr eigenes Gegengewinde in die Bohrung 23. Bei Ausgestaltungen mit nicht selbstfurchendem Gewinde ist das Gegengewinde vorgeschnitten. Der Schraubenkopf 43 ist axial auf der Schraubenaufnahme 16 abgestützt.

Bei nicht gezeigten Ausführungsformen ist die Schraube 40 mit einer Unterlegscheibe versehen, die form- und/oder kraftschlüssig mit einer Unterseite des Schraubenkopfs 43 verbunden ist.

Der axiale Abstand B entspricht vorliegend einer axialen Länge des Domschafts 22 (siehe insbesondere Fig. 3). Hierdurch ist ein Stirnende 25 des Schraubdoms 21 axial auf der Schraubenaufnahme 16 abgestützt, sodass sich weder zwischen dem Stirnende 25 des Schraubdoms 21 und der Schraubenaufnahme 16 noch zwischen der Außenseite 12 des ersten Bauteils 10 und einer Innenseite 26 des zweiten Bauteils 20 im Bereich außerhalb der Aussparung 13 ein Axialspalt bildet. Bei einer nicht gezeigten Ausführungsform ist der axiale Abstand B größer als die Länge des Domschafts 22, wodurch sich zwischen dem Stirnende 25 des Schraubdoms 21 und der Schraubenaufnahme 16 ein Axialspalt bildet. Hierdurch wird das zweite Bauteil 20 axial gegen die Außenseite 12 des ersten Bauteils 10 gezogen.

Bei den gezeigten Ausführungsformen ist das erste Bauteil 10 ein Blechbauteil 101. Dabei ist die Schraubenaufnahme 16 an einer Lasche 14 angeordnet. Die Lasche 14 ist durch einen abgekanteten Abschnitt 141 des Blechbauteils 101 ausgebildet.

Die Schraubenaufnahme 16 ist mit mindestens einer Ausnehmung 17 zur Aufnahme des Schraubenschafts 41 versehen. Bei der in Fig. 4 gezeigten Ausführungsform ist die Schraubenaufnahme 16 mit drei Ausnehmungen 17 versehen. Bei der in Fig. 5 gezeigten Ausführungsform ist jede der Schraubenaufnahmen 16 mit einer einzelnen Ausnehmung 17 versehen. Bei nicht gezeigten Ausführungsformen ist jede Schraubenaufnahme 16 mit zwei oder mehr als drei Ausnehmungen 17 versehen.

Bei den gezeigten Ausführungsformen ist die Ausnehmung 17 radial offen. Bei nicht gezeigten Ausführungsformen ist die Ausnehmung 17 radial geschlossen.

Das erste Bauteil 10 weist bei der gezeigten Ausführungsform mehrere Positionierelemente 19 und das zweite Bauteil 20 mehrere komplementäre Positionieraufnahmen 24 auf. Dabei greifen die Positionierelemente 19 jeweils in eine der Positionieraufnahmen 24 formschlüssig ein. Hierdurch sind die beiden Bauteile 10, 20 in vorbestimmter Lage aneinander ausgerichtet. Bei nicht gezeigten Ausführungsformen weist das zweite Bauteil 20 mehrere Positionierelemente und das erste Bauteil 10 mehrere komplementäre Positionieraufnahmen auf.

## Patentansprüche

1. Bauteilanordnung (A) für ein medizinisches Gerät (1), insbesondere ein Dialysegerät (2), aufweisend
ein erstes Bauteil (10) mit einer Innenseite (11), einer Außenseite (12) und einer Aussparung (13), die zwischen der Innenseite (11) und der Außenseite (12) erstreckt ist, und mit mindestens einer Schraubenaufnahme (16), die in der Aussparung (13) angeordnet ist und in Richtung der Innenseite (12) um einen axialen Abstand (B) von der Außenseite (12) zurückversetzt ist,
ein zweites Bauteil (20), das einen Schraubdom (21) mit einem längserstreckten Domschaft (22) und einer Bohrung (23) aufweist, wobei das zweite Bauteil (20) an der Außenseite (12) des ersten Bauteils (10) angeordnet ist und der Domschaft (22) axial in die Aussparung (13) hineinragt,
und eine Schraube (40) mit einem Schraubenschaft (41), der ein Gewinde (42) aufweist, und mit einem Schraubenkopf (43), wobei der Schraubenschaft (41) in die Bohrung (23) eingeschraubt ist und der Schraubenkopf (43) axial auf der Schraubenaufnahme (16) abgestützt ist, wodurch das erste Bauteil (10) mit dem zweiten Bauteil (20) verschraubt ist.

2. Bauteilanordnung (A) nach Anspruch 1, wobei der axiale Abstand (B) wenigstens im Wesentlichen einer axialen Länge des Domschafts (22) entspricht.

3. Bauteilanordnung (A) nach Anspruch 1 oder 2, wobei das erste Bauteil (10) ein Blechbauteil (101) ist und die mindestens eine Schraubenaufnahme (16) an einer Lasche (14) angeordnet ist, wobei die Lasche (14) durch einen abgekanteten Abschnitt (141) des Blechbauteils (101) ausgebildet ist.

4. Bauteilanordnung (A) nach einem der vorhergehenden Ansprüche, wobei die Schraubenaufnahme (16) mit mindestens einer Ausnehmung (17) zur Aufnahme des Schraubenschafts (41) versehen ist.

5. Bauteilanordnung (A) nach einem der vorhergehenden Ansprüche, wobei das erste Bauteil (10) mit mindestens zwei Schraubenaufnahmen (16) versehen ist, wobei die Schraubenaufnahmen (16) an mindestens zwei, insbesondere gegenüberliegenden, Umfangsseiten (18) der Aussparung (13) ausgebildet sind.

6. Bauteilanordnung (A) nach einem der vorhergehenden Ansprüche, wobei das erste Bauteil (10) mehrere Positionierelemente (19) und das zweite Bauteil (20) mehrere komplementäre Positionieraufnahmen (24) aufweist oder umgekehrt, wobei die Positionierelemente (19) jeweils in eine der Positionieraufnahmen (24) formschlüssig eingreifen, wodurch die beiden Bauteile (10, 20) in vorbestimmter Lage aneinander ausgerichtet sind.

7. Bauteilanordnung (A) nach einem der vorhergehenden Ansprüche, wobei das zweite Bauteil (20) aus einem Kunststoffmaterial (K) gefertigt, insbesondere spritzgegossen, ist.

8. Medizinisches Gerät (1), insbesondere Dialysegerät (2), mit einer Bauteilanordnung (A) nach einem der vorhergehenden Ansprüche.

## Claims

1. Component arrangement (A) for a medical device (1), in particular a dialysis device (2), comprising
a first component (10) having an inner side (11), an outer side (12) and a cutout (13) extending between the inner side (11) and the outer side (12), and having at least one screw receptacle (16) arranged in the cutout (13) and set back from the outer side (12) by an axial distance (B) in the direction of the inner side (12),
a second component (20) having a screw dome (21) with an elongate dome shaft (22) and a bore (23), wherein the second component (20) is arranged on the outer side (12) of the first component (10) and the dome shaft (22) protrudes axially into the cutout (13),
and a screw (40) with a screw shank (41), which has a thread (42), and with a screw head (43), wherein the screw shank (41) is screwed into the bore (23) and the screw head (43) is axially supported on the screw receptacle (16), as a result of which the first component (10) is screwed to the second component (20).

2. Component arrangement (A) according to Claim 1, wherein the axial distance (B) corresponds at least substantially to an axial length of the dome shaft (22).

3. Component arrangement (A) according to Claim 1 or 2, wherein the first component (10) is a sheet metal component (101) and the at least one screw receptacle (16) is arranged on a tab (14), wherein the tab (14) is formed by a bent-over portion (141) of the sheet metal component (101).

4. Component arrangement (A) according to any one of the preceding claims, wherein the screw receptacle (16) is provided with at least one recess (17) for receiving the screw shank (41).

5. Component arrangement (A) according to any one of the preceding claims, wherein the first component (10) is provided with at least two screw receptacles (16), wherein the screw receptacles (16) are formed on at least two, in particular opposite, circumferential sides (18) of the cutout (13).

6. Component arrangement (A) according to any one of the preceding claims, wherein the first component (10) has a plurality of positioning elements (19) and the second component (20) has a plurality of complementary positioning receptacles (24), or vice versa, wherein the positioning elements (19) each engage positively in one of the positioning receptacles (24), as a result of which the two components (10, 20) are aligned with each other in a predetermined position.

7. Component arrangement (A) according to any one of the preceding claims, wherein the second component (20) is produced, in particular injection moulded, from a plastics material (K).

8. Medical device (1), in particular a dialysis device (2), having a component arrangement (A) according to any one of the preceding claims.

## Revendications

1. Ensemble de composants (A) pour un appareil médical (1), en particulier un appareil de dialyse (2), comportant
un premier composant (10) avec une face intérieure (11), une face extérieure (12) et un évidement (13), qui s'étend entre la face intérieure (11) et la face extérieure (12), et avec au moins un logement de vis (16), qui est disposé dans l'évidement (13) et est en retrait en direction de la face intérieure (12) d'une distance axiale (B) par rapport à la face extérieure (12),
un deuxième composant (20), qui comporte un dôme de vis (21) avec une tige de dôme allongée (22) et un alésage (23), le deuxième composant (20) étant disposé sur la face extérieure (12) du premier composant (10) et la tige de dôme (22) faisant saillie axialement dans l'évidement (13),
et une vis (40) avec une tige de vis (41), qui comporte un filetage (42), et avec une tête de vis (43), la tige de vis (41) étant vissée dans l'alésage (23) et la tête de vis (43) prenant appui axialement sur le logement de vis (16), de sorte que le premier composant (10) est vissé au deuxième composant (20).

2. Ensemble de composants (A) selon la revendication 1, la distance axiale (B) correspondant au moins sensiblement à une longueur axiale de la tige de dôme (22).

3. Ensemble de composants (A) selon la revendication 1 ou 2, le premier composant (10) étant un composant en tôle (101) et l'au moins un logement de vis (16) étant disposé sur une languette (14), la languette (14) étant formée par une section coudée (141) du composant en tôle (101).

4. Ensemble de composants (A) selon l'une des revendications précédentes, le logement de vis (16) étant pourvu d'au moins un évidement (17) pour recevoir la tige de vis (41).

5. Ensemble de composants (A) selon l'une des revendications précédentes, le premier composant (10) étant pourvu d'au moins deux logements de vis (16), les logements de vis (16) étant formés sur au moins deux côtés périphériques (18), en particulier opposés, de l'évidement (13).

6. Ensemble de composants (A) selon l'une des revendications précédentes, le premier composant (10) comportant plusieurs éléments de positionnement (19) et le deuxième composant (20) comportant plusieurs logements de positionnement complémentaires (24) ou inversement, les éléments de positionnement (19) venant en prise chacun par complémentarité de forme avec l'un des logements de positionnement (24), ce qui permet d'aligner les deux composants (10, 20) l'une sur l'autre dans une position prédéterminée.

7. Ensemble de composants (A) selon l'une des revendications précédentes, le deuxième composant (20) étant fabriqué, en particulier moulé par injection, à partir d'un matériau en matière plastique (K).

8. Appareil médical (1), en particulier appareil de dialyse (2), avec un ensemble de composants (A) selon l'une des revendications précédentes.
